# FASCICULE DE BREVET EUROPEEN

(11) **EP 2 486 092 B1**
(45) Date de publication et mention de la délivrance du brevet: **14.08.2013**
(21) Numéro de dépôt: 10762924.8
(22) Date de dépôt: 08.10.2010
(51) Int. Cl.: C08K 5/46, C08K 5/47, B60C 1/00, C08K 5/00, C07D 285/04

(54) **COMPOSITION DE CAOUTCHOUC COMPRENANT UN THIADIAZOLE**
KAUTSCHUKZUSAMMENSETZUNG MIT THIADIAZOLVERBINDUNG
RUBBER COMPOSITION COMPRISING A THIADIAZOL COMPOUND

(30) Priorité: 08.10.2009 FR 0957039
(43) Date de publication de la demande: 15.08.2012
(73) Titulaire: COMPAGNIE GENERALE DES ETABLISSEMENTS MICHELIN, 63000 Clermont-Ferrand (FR); MICHELIN Recherche et Technique S.A., 1763 Granges-Paccot (CH)
(72) Inventeur: VEYLAND, Anne, F-63200 Marsat (FR); SEEBOTH, Nicolas, F-63000 Clermont-Ferrand (FR); ARAUJO DA SILVA, José Carlos, F-63430 Pont Du Chateau (FR)
(74) Mandataire: Sidhu, Alban
(86) Numéro de dépôt international: PCT/EP2010/065072
(87) Numéro de publication internationale: WO 2011/042525

(56) Documents cités:
- JP-A- 9 012 782
- US-A- 3 899 502
- US-A1- 2004 176 504

## Description

La présente invention se rapporte à une composition de caoutchouc utilisable notamment pour la fabrication de pneumatiques ou de produits semi-finis pour pneumatiques tels que des bandes de roulement, ladite composition étant à base d'un élastomère diénique, d'une charge renforçante et d'un système de vulcanisation comprenant un composé thiadiazole particulier.

La vulcanisation des élastomères diéniques par le soufre est largement utilisée dans l'industrie du caoutchouc, en particulier celle du pneumatique. Pour vulcaniser les élastomères diéniques, on utilise un système de vulcanisation relativement complexe comportant, en plus du soufre, un accélérateur primaire de vulcanisation, tels que les sulfénamides à noyau benzothiazole, ainsi que divers accélérateurs secondaires ou activateurs de vulcanisation, tout particulièrement des dérivés du zinc tels que l'oxyde de zinc (ZnO) seul ou utilisé avec des acides gras.

Les sulfénamides à noyau benzothiazole utilisés comme accélérateurs primaire de vulcanisation sont par exemple la N-cyclohexyl-2-benzothiazyle sulfénamide (en abrégé « CBS »), la N,N-dicyclohexyl-2-benzothiazyle sulfénamide (en abrégé « DCBS »), la N-ter-butyl-2-benzothiazyle sulfénamide (en abrégé « TBBS ») et les mélanges de ces composés.

Les compositions de caoutchouc doivent présenter une réticulation suffisante tout en conservant un compromis acceptable entre les différentes propriétés rhéométriques.

La demanderesse a découvert une nouvelle composition de caoutchouc comprenant à titre d'accélérateur de vulcanisation un nouveau composé thiadiazole. Cette nouvelle composition de caoutchouc permet d'obtenir un compromis de propriétés rhéométriques similaire à celui obtenu avec des compositions de caoutchouc contenant des accélérateurs de vulcanisation classiquement utilisés.

L'invention a donc pour objet une composition de caoutchouc pour la fabrication de pneumatiques, à base d'un ou plusieurs élastomères diéniques, d'une ou plusieurs charges renforçantes et d'un système de vulcanisation, ledit système de vulcanisation comprenant un ou plusieurs composés thiadiazoles de formule 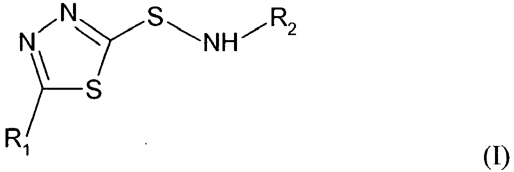 où
R₁ représente indépendamment H ou un groupe hydrocarboné en C₁-C₂₅ choisi parmi les groupes alkyles linéaires, ramifiés ou cycliques, et les groupes aryles, éventuellement interrompus par un ou plusieurs hétéroatomes,
R₂ représente :
- un groupe alkyle en C₁-C₂₅, linéaire ou ramifié, éventuellement interrompu par un ou plusieurs hétéroatomes, éventuellement substitué par un ou plusieurs groupes alkyles cycliques en C₁-C₁₀ ou aryles en C₆-C₁₂, ou
- un groupe alkyle cyclique en C₃-C₁₀, éventuellement interrompu par un ou plusieurs hétéroatomes, éventuellement substitué par un ou plusieurs groupes alkyles linéaires, ramifiés ou cycliques en C₁-C₂₅ ou aryles en C₆-C₁₂, éventuellement interrompus par un ou plusieurs hétéroatomes.

L'invention a également pour objet un procédé pour préparer une composition de caoutchouc pour la fabrication de pneumatiques telle que définie à l'une quelconque des revendications précédentes caractérisé en ce qu'il comprend les étapes suivantes :
- incorporer à ou aux élastomères diéniques, au cours d'une première étape dite « non-productive », la ou les charges renforçantes, en malaxant thermomécaniquement le tout, en une ou plusieurs fois, jusqu'à atteindre une température maximale comprise entre 130°C et 200°C,
- refroidir l'ensemble à une température inférieure à 100°C,
- incorporer ensuite, au cours d'une seconde étape dite « productive », le système de vulcanisation, puis
- malaxer le tout jusqu'à une température maximale inférieure à 120°C.

L'invention a encore pour objet l'utilisation d'une composition selon l'invention pour la fabrication d'un article fini ou d'un produit semi-fini destiné à un système de liaison au sol de véhicule automobile, tel que pneumatique, appui interne de sécurité pour pneumatique, roue, ressort en caoutchouc, articulation élastomérique, autre élément de suspension et anti-vibratoire. En particulier, la composition selon l'invention peut être utilisée pour la fabrication de produits semi-finis en caoutchouc destinés à des pneumatiques, tel que les bandes de roulement, les nappes d'armature de sommet, les flancs, les nappes d'armature de carcasse, les bourrelets, les protecteurs, les sous-couches, les blocs de caoutchouc et autres gommes internes, notamment les gommes de découplage, destinés à assurer la liaison ou l'interface entre les zones précitées des pneumatiques.

L'invention a encore pour objet un article fini ou produit semi-fini destiné à un système de liaison au sol de véhicule automobile, en particulier les pneumatiques et produits semi-finis pour pneumatiques, comprenant une composition selon l'invention. Les pneumatiques conformes à l'invention sont notamment destinés à des véhicules tourisme comme à des véhicules industriels choisis parmi camionnettes, « Poids-lourd »- i.e. métro, bus, engins de transport routier (camions, tracteurs, remorques), véhicules hors-la-route -, engin agricoles ou de génie civil, avions, autres véhicules de transport ou de manutention.

L'invention a enfin pour objet un l'utilisation comme accélérateur de vulcanisation dans une composition à base d'un ou plusieurs élastomères diéniques, d'une ou plusieurs charges renforçantes et d'un système de vulcanisation, d'un ou plusieurs composés thiadiazoles de formule (I).

L'invention ainsi que ses avantages seront aisément compris à la lumière de la description et des exemples de réalisation qui suivent.

### 1. Mesures et tests utilisés

Les compositions de caoutchouc, dans lesquelles sont testés les accélérateurs de vulcanisation thiadiazoles, sont caractérisées, avant et après cuisson, comme indiqué ci-après.

### Rhéométrie

Les mesures sont effectuées à 150°C avec un rhéomètre à chambre oscillante, selon la norme DIN 53529 - partie 3 (juin 1983). L'évolution du couple rhéométrique, ΔCouple, en fonction du temps décrit l'évolution de la rigidification de la composition par suite de la réaction de vulcanisation. Les mesures sont traitées selon la norme DIN 53529 - partie 2 (mars 1983) : T₀ est le délai d'induction, c'est-à-dire le temps nécessaire au début de la réaction de vulcanisation ; T_{α} (par exemple T₉₉) est le temps nécessaire pour atteindre une conversion de α%, c'est-à-dire α% (par exemple 99%) de l'écart entre les couples minimum et maximum. On mesure également la constante de vitesse de conversion notée K (exprimée en min-1), d'ordre 1, calculée entre 30% et 80% de conversion, qui permet d'apprécier la cinétique de vulcanisation.

### II. Conditions de réalisation de l'invention

Comme expliqué précédemment, la composition selon l'invention est à base d'un ou plusieurs élastomères diéniques, d'une ou plusieurs charges renforçantes et d'un système de vulcanisation.

Par l'expression composition « à base de », il faut entendre une composition comportant le mélange et/ou le produit de réaction des différents constituants utilisés, certains de ces constituants de base étant susceptibles de, ou destinés à réagir entre eux, au moins en partie, lors des différentes phases de fabrication de la composition, en particulier au cours de sa vulcanisation.

Dans la présente description, sauf indication expresse différente, tous les pourcentages (%) sont des % en masse. D'autre part, tout intervalle de valeurs désigné par l'expression "entre a et b" représente le domaine de valeurs allant de plus de a à moins de b (c'est-à-dire bornes a et b exclues) tandis que tout intervalle de valeurs désigné par l'expression "de a à b" signifie le domaine de valeurs allant de a jusqu'à b (c'est-à-dire incluant les bornes strictes a et b).

### II-1. Elastomère diénique

Par élastomère ou caoutchouc "diénique", doit être compris de manière connue un élastomère issu au moins en partie (i.e., un homopolymère ou un copolymère) de monomères diènes (monomères porteurs de deux doubles liaisons carbone-carbone, conjuguées ou non).

Ces élastomères diéniques peuvent être classés dans deux catégories : "essentiellement insaturés" ou "essentiellement saturés". On entend en général par "essentiellement insaturé", un élastomère diénique issu au moins en partie de monomères diènes conjugués, ayant un taux de motifs ou unités d'origine diénique (diènes conjugués) qui est supérieur à 15% (% en moles) ; c'est ainsi que des élastomères diéniques tels que les caoutchoucs butyle ou les copolymère de diènes et d'alpha-oléfines type EPDM n'entrent pas dans la définition précédente et peuvent être notamment qualifiés d'élastomères diéniques "essentiellement saturés" (taux de motifs d'origine diénique faible ou très faible, toujours inférieur à 15%). Dans la catégorie des élastomères diéniques "essentiellement insaturés", on entend en particulier par élastomère diénique "fortement insaturé" un élastomère diénique ayant un taux de motifs d'origine diénique (diènes conjugués) qui est supérieur à 50%.

Ces définitions étant données, on entend plus particulièrement par élastomère diénique susceptible d'être utilisé dans les compositions conformes à l'invention:
(a) - tout homopolymère obtenu par polymérisation d'un monomère diène conjugué ayant de 4 à 12 atomes de carbone;
(b) - tout copolymère obtenu par copolymérisation d'un ou plusieurs diènes conjugués entre eux ou avec un ou plusieurs composés vinyle aromatique ayant de 8 à 20 atomes de carbone;
(c) - un copolymère ternaire obtenu par copolymérisation d'éthylène, d'une α-oléfine ayant 3 à 6 atomes de carbone avec un monomère diène non conjugué ayant de 6 à 12 atomes de carbone, comme par exemple les élastomères obtenus à partir d'éthylène, de propylène avec un monomère diène non conjugué du type précité tel que notamment l'hexadiène-1,4, l'éthylidène norbornène, le dicyclopentadiène;
(d) - un copolymère d'isobutène et d'isoprène (caoutchouc butyle), ainsi que les versons halogénées, en particulier chlorées ou bromées, de ce type de copolymère.

Bien qu'elle s'applique à tout type d'élastomère diénique, l'homme du métier du pneumatique comprendra que la présente invention est de préférence mise en oeuvre avec des élastomères diéniques essentiellement insaturés, en particulier du type (a) ou (b) ci-dessus.

A titre de diènes conjugués conviennent notamment le butadiène-1,3, le 2-méthyl-1,3-butadiène, les 2,3-di(alkyle en C₁-C₅)-1,3-butadiènes tels que par exemple le 2,3-diméthyl-1,3-butadiène, le 2,3-diéthyl-1,3-butadiène, le 2-méthyl-3-éthyl-1,3-butadiène, le 2-méthyl-3-isopropyl-1,3-butadiène, un aryl-1,3-butadiène, le 1,3-pentadiène, le 2,4-hexadiène. A titre de composés vinylaromatique conviennent par exemple le styrène, l'ortho-, méta-, para-méthylstyrène, le mélange commercial "vinyle-toluène", le para-tertiobutylstyrène, les méthoxystyrènes, les chlorostyrénes, le vinylmésitylène, le divinylbenzène, le vinylnaphtalène.

Les copolymères peuvent contenir entre 99% et 20% en poids d'unités diéniques et entre 1% et 80% en poids d'unités vinylaromatique. Les élastomères peuvent avoir toute microstructure qui est fonction des conditions de polymérisation utilisées, notamment de la présence ou non d'un agent modifiant et/ou randomisant et des quantités d'agent modifiant et/ou randomisant employées. Les élastomères peuvent être par exemple à blocs, statistiques, séquencés, microséquencés, et être préparés en dispersion, en émulsion ou en solution ; ils peuvent être couplés et/ou étoilés ou encore fonctionnalisés avec un agent de couplage et/ou d'étoilage ou de fonctionnalisation. Pour un couplage à du noir de carbone, on peut citer par exemple des groupes fonctionnels comprenant une liaison C-Sn ou des groupes fonctionnels aminés tels que aminobenzophénone par exemple ; pour un couplage à une charge inorganique renforçante telle que silice, on peut citer par exemple des groupes fonctionnels silanol ou polysiloxane ayant une extrémité silanol (tels que décrits par exemple dans FR 2 740 778,US 6 013 718 ou WO 2008/141702), des groupes alkoxysilane (tels que décrits par exemple dans FR 2 765 882 ou US 5 977 238), des groupes carboxyliques (tels que décrits par exemple dans WO 01/92402 ou US 6 815 473, WO 2004/096865 ou US 2006/0089445) ou encore des groupes polyéthers (tels que décrits par exemple dans EP 1 127 909, US 6 503 973 WO 2009/000750 ou WO 2009/000752). Comme autres exemples d'élastomères fonctionnalisés, on peut citer également des élastomères (tels que SBR, BR, NR ou IR) du type époxydés.

Conviennent les polybutadiènes et en particulier ceux ayant une teneur (% molaire) en unités -1,2 comprise entre 4% et 80% ou ceux ayant une teneur (% molaire) en cis-1,4 supérieure à 80%, les polyisoprènes, les copolymères de butadiène-styrène et en particulier ceux ayant une Tg (température de transition vitreuse (Tg, mesurée selon ASTM D3418) comprise entre 0°C et - 70°C et plus particulièrement entre - 10°C et - 60°C, une teneur en styrène comprise entre 5% et 60% en poids et plus particulièrement entre 20% et 50%, une teneur (% molaire) en liaisons -1,2 de la partie butadiénique comprise entre 4% et 75%, une teneur (% molaire) en liaisons trans-1,4 comprise entre 10% et 80%, les copolymères de butadiène-isoprène et notamment ceux ayant une teneur en isoprène comprise entre 5% et 90% en poids et une Tg de - 40°C à - 80°C, les copolymères isoprène-styrène et notamment ceux ayant une teneur en styrène comprise entre 5% et 50% en poids et une Tg comprise entre 5°C et - 50°C. Dans le cas des copolymères de butadiène-styrène-isoprène conviennent notamment ceux ayant une teneur en styrène comprise entre 5% et 50% en poids et plus particulièrement comprise entre 10% et 40%, une teneur en isoprène comprise entre 15% et 60% en poids et plus particulièrement entre 20% et 50%, une teneur en butadiène comprise entre 5% et 50% en poids et plus particulièrement comprise entre 20% et 40%, une teneur (% molaire) en unités -1,2 de la partie butadiénique comprise entre 4% et 85%, une teneur (% molaire) en unités trans -1,4 de la partie butadiénique comprise entre 6% et 80%, une teneur (% molaire) en unités -1,2 plus -3,4 de la partie isoprénique comprise entre 5% et 70% et une teneur (% molaire) en unités trans -1,4 de la partie isoprénique comprise entre 10% et 50%, et plus généralement tout copolymère butadiène-styrène-isoprène ayant une Tg comprise entre - 5°C et - 70°C.

En résumé, le ou les élastomères diéniques de la composition selon l'invention sont choisis préférentiellement dans le groupe des élastomères diéniques fortement insaturés constitué par les polybutadiènes (en abrégé "BR"), les polyisoprènes (IR) de synthèse, le caoutchouc naturel (NR), les copolymères de butadiène, les copolymères d'isoprène et les mélanges de ces élastomères. De tels copolymère sont plus préférentiellement choisis dans le groupe constitué par les copolymères de butadiène-styrène (SBR), les copolymères d'isoprène-butadiène (BIR), les copolymères d'isoprène-styrène (SIR) et les copolymères d'isoprène-butadiène-styrène (SBIR).

De préférence, l'élastomère diénique est le caoutchouc naturel.

Selon un mode de réalisation particulier, l'élastomère diénique est majoritairement (i.e., pour plus de 50 pcc) un SBR, qu'il s'agisse d'un SBR préparé en émulsion ("ESBR") ou d'un SBR préparé en solution ("SSBR"), ou un coupage (mélange) SBR/BR, SBR/NR (ou SBR/IR), BR/NR (ou BR/IR), ou encore SBR/BR/NR (ou SBR/BR/IR). Dans le cas d'un élastomère SBR (ESBR ou SSBR), on utilise notamment un SBR ayant une teneur en styrène moyenne, par exemple comprise entre 20% et 35% en poids, ou une teneur en styrène élevée, par exemple de 35 à 45%, une teneur en liaisons vinyliques de la partie butadiénique comprise entre 15% et 70%, une teneur (% molaire) en liaisons trans-1,4 comprise entre 15% et 75% et une Tg comprise entre - 10°C et - 55°C ; un tel SBR peut être avantageusement utilisé en mélange avec un BR possédant de préférence plus de 90% (% molaire) de liaisons cis-1,4.

Selon un autre mode de réalisation particulier, l'élastomère diénique est majoritairement (pour plus de 50 pce) un élastomère isoprénique. C'est le cas en particulier lorsque les compositions de l'invention sont destinées à constituer, dans les pneumatiques, les matrices de caoutchouc de certaines bandes de roulement (par exemple pour véhicules industriels), de nappes d'armature de sommet (par exemple de nappes de travail, nappes de protection ou nappes de frettage), de nappes d'armature de carcasse, de flancs, de bourrelets, de protecteurs, de sous-couches, de blocs de caoutchouc et autres gommes internes assurant l'interface entre les zones précitées des pneumatiques.

Par "élastomère isoprénique", on entend de manière connue un homopolymère ou un copolymère d'isoprène, en d'autres termes un élastomère diénique choisi dans le groupe constitué par le caoutchouc naturel (NR), les polyisoprènes de synthèse (IR), les différents copolymères d'isoprène et les mélanges de ces élastomères. Parmi les copolymères d'isoprène, on citera en particulier les copolymères d'isobuténe-isoprène (caoutchouc butyle - IIR), d'isoprène-styrène (SIR), d'isoprène-butadiène (BIR) ou d'isoprène-butadiène-styrène (SBIR). Cet élastomère isoprénique est de préférence du caoutchouc naturel ou un polyisoprène cis-1,4 de synthèse; parmi ces polyisoprènes de synthèse, sont utilisés de préférence des polyisoprènes ayant un taux (% molaire) de liaisons cis-1,4 supérieur à 90%. plus préférentiellement encore supérieur à 98%.

Selon un autre mode de réalisation particulier, notamment lorsqu'elle est destinée à un flanc de pneumatique, à une gomme intérieure étanche de pneumatique sans chambre (ou autre élément imperméable à l'air), la composition conforme à l'invention peut contenir au moins un élastomère diénique essentiellement saturé, en particulier au moins un copolymère EPDM ou un caoutchouc butyle (éventuellement chloré ou bromé), que ces copolymères soient utilisés seuls ou en mélange avec des élastomères diénique fortement insaturés tels que cités précédemment, notamment NR ou IR, BR ou SBR.

Selon un autre mode préférentiel de réalisation de l'invention, la composition de caoutchouc comprend un coupage d'un (un ou plusieurs) élastomère diénique dit "à haute Tg" présentant une Tg comprise entre - 70°C et 0°C et d'un (un ou plusieurs) élastomère diénique dit "à basse Tg" comprise entre -110°C et -80°C, plus préférentiellement entre -105°C et -90°C. L'élastomère à haute Tg est choisi de préférence dans le groupe constitué par les S-SBR, les E-SBR, le caoutchouc naturel, les polyisoprènes de synthèse (présentant un taux (% molaire) d'enchaînements cis-1,4 de préférence supérieur à 95%), les BIR, les SIR, les SBIR, et les mélanges de ces élastomères. L'élastomère à basse Tg comprend de préférence des unités butadiène selon un taux (% molaire) au moins égal à 70% ; il consiste de préférence en un polybutadiène (BR) présentant un taux (% molaire) d'enchaînements cis-1,4 supérieur à 90%.

Selon un autre mode particulier de réalisation de l'invention, la composition de caoutchouc comprend par exemple de 30 à 100 pce, en particulier de 50 à 100 pce, d'un élastomère à haute Tg en coupage avec 0 à 70 pce, en particulier de 0 à 50 pce, d'un élastomère à basse Tg ; selon un autre exemple, elle comporte pour la totalité des 100 pce un ou plusieurs SBR préparé(s) en solution.

Selon un autre mode particulier de réalisation de l'invention, l'élastomère diénique de la composition selon l'invention comprend un coupage d'un BR (à titre d'élastomère basse Tg) présentant un taux (% molaire) d'enchaînements cis-1,4 supérieur à 90%, avec un ou plusieurs S-SBR ou E-SBR (à titre d'élastomère(s) haute Tg).

La composition selon l'invention peut contenir un seul élastomère diénique ou un mélange de plusieurs élastomères diéniques, le ou les élastomères diéniques pouvant être utilisés en association avec tout type d'élastomère synthétique autre que diénique, voire avec des polymères autres que des élastomères, par exemple des polymères thermoplastiques.

### II-2. Charge renforçante

On peut utiliser tout type de charge renforçante connue pour ses capacités à renforcer une composition de caoutchouc utilisable pour la fabrication de pneumatiques, par exemple une charge organique tel que du noir de carbone, une charge inorganique renforçante telle que de la silice, ou encore un coupage de ces deux types de charge, notamment un coupage de noir de carbone et de silice.

Comme noirs de carbone conviennent tous les noirs de carbone, notamment les noirs du type HAF, ISAF, SAF conventionnellement utilisés dans les pneumatiques (noirs dits de grade pneumatique). Parmi ces derniers, on citera plus particulièrement les noirs de carbone renforçants des séries 100, 200 ou 300 (grades ASTM), comme par exemple les noirs N115, N134, N234, N326, N330, N339, N347, N375, ou encore, selon les applications visées, les noirs de séries plus élevées (par exemple N660, N683, N772). Les noirs de carbone pourraient être par exemple déjà incorporés à l'élastomère isoprénique sous la forme d'un masterbatch (voir par exemple demandes WO 97/36724 ou WO 99/16600).

Comme exemples de charges organiques autres que des noirs de carbone, on peut citer les charges organiques de polyvinylaromatique fonctionnalisé telles que décrites dans les demandes WO-A-2006/069792 et WO-A-2006/069793.

Par "charge inorganique renforçante", doit être entendu dans la présente demande, par définition, toute charge inorganique ou minérale (quelles que soient sa couleur et son origine (naturelle ou de synthèse), encore appelée charge "blanche", charge "claire" voire "charge non noire" ("non-black filler") par opposition au noir de carbone, capable de renforcer à elle seule, sans autre moyen qu'un agent de couplage intermédiaire, une composition de caoutchouc destinée à la fabrication de pneumatiques, en d'autres termes apte à remplacer, dans sa fonction de renforcement, un noir de carbone conventionnel de grade pneumatique ; une telle charge se caractérise généralement, de manière connue, par la présence de groupes hydroxyle (-OH) à sa surface.

L'état physique sous lequel se présente la charge inorganique renforçante est indifférent, que ce soit sous forme de poudre, de microperles, de granulés, de billes ou toute autre forme densifiée appropriée. Bien entendu on entend également par charge inorganique renforçante des mélanges de différentes charges inorganiques renforçantes, en particulier de charges siliceuses et/ou alumineuses hautement dispersibles telles que décrites ci-après.

Comme charges inorganiques renforçantes conviennent notamment des charges minérales du type siliceuse, en particulier de la silice (SiO₂), ou du type alumineuse, en particulier de l'alumine (Al₂O₃). La silice utilisée peut être toute silice renforçante connue de l'homme du métier, notamment toute silice précipitée ou pyrogénée présentant une surface BET ainsi qu'une surface spécifique CTAB toutes deux inférieures à 450 m²/g, de préférence de 30 à 400 m²/g. A titres de silices précipitées hautement dispersibles (dites "HDS"), on citera par exemple les silices Ultrasil 7000 et Ultrasil 7005 de la société Degussa, les silices Zeosil 1165MP, 1135MP et 1115MP de la société Rhodia, la silice Hi-Sil EZ150G de la société PPG, les silices Zeopol 8715, 8745 et 8755 de la Société Huber, les silices à haute surface spécifique telles que décrites dans la demande WO 03/16837.

Lorsque la composition selon l'invention est destinée à des bandes de roulement de pneumatique à faible résistance au roulement, la charge inorganique renforçante utilisée, en particulier s'il s'agit de silice, a de préférence une surface BET comprise entre 45 et 400 m²/g, plus préférentiellement comprise entre 60 et 300 m²/g.

De manière préférentielle, le taux de charge renforçante totale (noir de carbone et/ou charge inorganique renforçante telle que silice) est compris entre 20 et 200 pce, plus préférentiellement entre 30 et 150 pce, l'optimum étant de manière connue différent selon les applications particulières visées : le niveau de renforcement attendu sur un pneumatique vélo, par exemple, est bien sûr inférieur à celui exigé sur un pneumatique apte à rouler à grande vitesse de manière soutenue, par exemple un pneu moto, un pneu pour véhicule de tourisme ou pour véhicule utilitaire tel que Poids lourd.

Selon un mode de réalisation de l'invention, on utilise une charge renforçante comportant entre 30 et 150 pce, plus préférentiellement entre 50 et 120 pce de charge inorganique, particulièrement de silice, et optionnellement du noir de carbone ; le noir de carbone, lorsqu'il est présent, est utilisé de préférence à un taux inférieur à 20 pce, plus préférentiellement inférieur à 10 pce (par exemple entre 0,1 et 10 pce).

Selon un autre mode de réalisation, la charge renforçante est le noir de carbone.

Pour coupler la charge inorganique renforçante à l'élastomère diénique, on utilise de manière connue un agent de couplage (ou agent de liaison) au moins bifonctionnel destiné à assurer une connexion suffisante, de nature chimique et/ou physique, entre la charge inorganique (surface de ses particules) et l'élastomère diénique, en particulier des organosilanes ou des polyorganosiloxanes bifonctionnels.

On utilise notamment des silanes polysulfurés, dits "symétriques" ou "asymétriques" selon leur structure particulière, tels que décrits par exemple dans les demandes WO03/002648 (ou US 2005/016651) et WO03/002649 (ou US 2005/016650).

Conviennent en particulier, sans que la définition ci-après soit limitative, des silanes polysulfurès dits "asymétriques" répondant à la formule générale (III) suivante:

(III) Z - A- Sₓ - A - Z ,

dans laquelle:
- x est un entier de 2 à 8 (de préférence de 2 à 5) ;
- A est un radical hydrocarboné divalent (de préférence des groupements alkylène en C₁-C₁₈ ou des groupements arylène en C₆-C₁₂, plus particulièrement des alkylènes en C₁-C₁₀, notamment en C₁-C₄, en particulier le propylène) ;
- Z répond à l'une des formules ci-après: 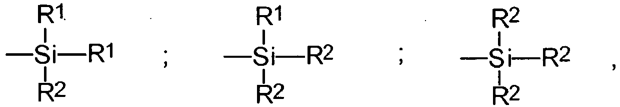 dans lesquelles:
- les radicaux R¹, substitués ou non substitués, identiques ou différents entre eux, représentent un groupe alkyle en C₁-C₁₈, cycloalkyle en C₅-C₁₈ ou aryle en C₆-C₁₈ (de préférence des groupes alkyle en C₁-C₆, cyclohexyle ou phényle, notamment des groupes alkyle en C₁-C₄, plus particulièrement le méthyle et/ou l'éthyle).
- les radicaux R², substitués ou non substitués, identiques ou différents entre eux, représentent un groupe alkoxyle en C₁-C₁₈ ou cycloalkoxyle en C₅-C₁₈ (de préférence un groupe choisi parmi alkoxyles en C₁-C₈ et cycloalkoxyles en C₅-C₈, plus préférentiellement encore un groupe choisi parmi alkoxyles en C₁-C₄, en particulier méthoxyle et éthoxyle).

Dans le cas d'un mélange d'alkoxysilancs polysulfurés répondant à la formule (III) ci-dessus, notamment des mélanges usuels disponibles commercialement, la valeur moyenne des "x" est un nombre fractionnaire de préférence compris entre 2 et 5, plus préférentiellement proche de 4. Mais l'invention peut être aussi avantageusement mise en oeuvre par exemple avec des alkoxysilanes disulfurés (x = 2).

A titre d'exemples de silanes polysulfurés, on citera plus particulièrement les polysulfures (notamment disulfures, trisulfures ou tétrasulfures) de bis-(alkoxyl(C₁-C₄)-alkyl(C₁-C₄)silyl-alkyl(C₁-C₄)), comme par exemple les polysulfures de bis(3-triméthoxysilylpropyl) ou de bis(3-triéthoxysilylpropyl). Parmi ces composés, on utilise en particulier, le tétrasulfure de bis(3-triéthoxysilylpropyl), en abrégé TESPT, de formule [(C₂H₅O)₃Si(CH₂)₃S₂]₂ ou le disulfure de bis-(triéthoxysilylpropyle), en abrégé TESPD, de formule [(C₂H₅O)₃Si(CH₂)₃S]₂. On citera également à titre d'exemples préférentiels les polysulfures (notamment disulfures, trisulfures ou tétrasulfures) de bis-(monoalkoxyl(C₁-C₄)-dialkyl(C₁-C₄)silylpropyl), plus particulièrement le tétrasulfure de bis-monoéthoxydiméthylsilylpropyl tel que décrit dans la demande de brevet WO 02/083782 (ou US 2004/132880).

A titre d'agent de couplage autre qu'alkoxysilane polysulfuré, on citera notamment des POS (polyorganosiloxanes) bifonctionnels ou encore des polysulfures d'hydroxysilane (R² = OH dans la formule III ci-dessus) tels que décrits dans les demandes de brevet WO 02/30939 (ou US 6,774,255) et WO 02/31041 (ou US 2004/051210), ou encore des silanes ou POS porteurs de groupements fonctionnels azo-dicarbonyle, tels que décrits par exemple dans les demandes de brevet WO 2006/125532, WO 2006/125533, WO 2006/125534.

Dans les compositions de caoutchouc conformes à l'invention, la teneur en agent de couplage est préférentiellement comprise entre 4 et 12 pce, plus préférentiellement entre 3 et 8 pce.

L'homme du métier comprendra qu'à titre de charge équivalente de la charge inorganique renforçante décrite dans le présent paragraphe, pourrait être utilisée une charge renforçante d'une autre nature, notamment organique, dès lors que cette charge renforçante serait recouverte d'une couche inorganique telle que silice, ou bien comporterait à sa surface des sites fonctionnels, notamment hydroxyles, nécessitant l'utilisation d'un agent de couplage pour établir la liaison entre la charge et l'élastomère.

### 11.3 Système de vulcanisation

Le système de vulcanisation proprement dit est à base de soufre (ou d'un agent donneur de soufre) et d'un accélérateur primaire de vulcanisation. A ce système de vulcanisation de base viennent s'ajouter, incorporés au cours de la première phase non-productive et/ou au cours de la phase productive telles que décrites ultérieurement, divers accélérateurs secondaires ou activateurs de vulcanisation connus tels qu'oxyde de zinc, acide stéarique ou composés équivalents, dérivés guanidiques (en particulier diphénylguanidine).

Le soufre est utilisé à un taux préférentiel compris entre 0,5 et 10 pce, plus préférentiellement compris entre 0,5 et 5 pce, en particulier entre 0,5 et 3 pce lorsque la composition de l'invention est destinée, selon un mode préférentiel de l'invention, à constituer une bande de roulement de pneumatique.

L'accélérateur primaire de vulcanisation doit permettre une réticulation des compositions de caoutchouc dans des temps industriellement acceptables, tout en préservant un délai minimum de sécurité (« temps de grillage ») au cours duquel les compositions peuvent être mises en forme sans risque de vulcanisation prématurée (« grillage »).

Selon l'invention, le système de vulcanisation comprend, à titre d'accélérateur primaire de vulcanisation, un ou plusieurs composés thiadiazoles de formule 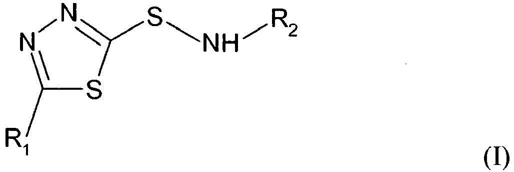 où
R₁ représente indépendamment H ou un groupe hydrocarboné en C₁-C₂₅ choisi parmi les groupes alkyles linéaires, ramifiés ou cycliques, et les groupes aryles, éventuellement interrompus par un ou plusieurs hétéroatomes,
R₂ représente :
- un groupe alkyle en C₁-C₂₅, linéaire ou ramifié, éventuellement interrompu par un ou plusieurs hétéroatomes, éventuellement substitué par un ou plusieurs groupes alkyles cycliques en C₃-C₁₀ ou aryles en C₆-C₁₂, ou
- un groupe alkyle cyclique en C₃-C₁₀, éventuellement interrompu par un ou plusieurs hétéroatomes, éventuellement substitué par un ou plusieurs groupes alkyles linéaires, ramifiés ou cycliques en C₁-C₂₅ ou aryles en C₆-C₁₂, éventuellement interrompus par un ou plusieurs hétéroatomes.

Les composés de formule (I) peuvent avantageusement remplacer en tout ou partie les composés sulfénamides classiquement utilisés.

Par groupe alkyle cyclique, on entend un groupe alkyle constitué d'un ou plusieurs cycles.

Le ou les hétéroatomes peuvent être un atome d'azote, de soufre ou d'oxygène.

De préférence, le composé thiadiazole de formule (I) est tel que :
R₁ représente H ou un groupe hydrocarboné en C₁-C₂₅ choisi parmi les groupes alkyles linéaires, ramifiés ou cycliques, et les groupes aryles, éventuellement interrompus par un ou plusieurs hétéroatomes,
R₂ représente :
   - un groupe alkyle linéaire en C₂-C₂₅ ou ramifié en C₃ ou C₅-C₂₅, éventuellement interrompu par un ou plusieurs hétéroatomes, éventuellement substitué par un ou plusieurs groupes alkyles cycliques en C₃-C₁₀ ou aryles en C₆-C₁₂, ou
   - un groupe alkyle cyclique en C₃-C₁₀, éventuellement interrompu par un ou plusieurs hétéroatomes, éventuellement substitué par un ou plusieurs groupes alkyles linéaires, ramifiés ou cycliques en C₁-C₂₅ ou aryles en C₆-C₁₂, éventuellement interrompus par un ou plusieurs hétéroatomes.

Selon un premier mode de réalisation, R₁ représente un groupe méthyle.

Selon un deuxième mode de réalisation, R₁ représente H.

Selon un mode de réalisation, R₂ représente avantageusement un groupe alkyle cyclique en C₃-C₁₀.

En particulier, R₂ peut représenter un groupe cyclohexyle.

Selon un autre mode de réalisation, R₂ représente un groupe tertbutyle.

De préférence, R₂ représente un groupe cyclohexyle.

Ainsi, un composé de formule (I) préféré est celui dans lequel R₁ représente H et R₂ représente un cyclohexyle. Dans ce cas, le composé thiadiazole de formule (I) est la N-cyclohexyl-S-(1,3,4-thiadiazol-2-yl)thiohydroxylamine.

Un autre composé de formule (I) préféré est celui dans lequel R₁ représente un méthyle et R₂ représente un cyclohexyle. Dans ce cas, le composé thiadiazole de formule (I) est la N-cyclohexyl-S-(5-méthyl-1,3,4-thiadiazol-2-yl)thiohydroxylamine.

Le ou les composés de formule (I) représentent généralement de 0,1 à 7 pce, de préférence de 0,5 à 7 pce, mieux de 0,5 à 5 pce.

Le ou les composés thiadiazoles présents dans la composition selon l'invention peuvent être préparés selon le procédé comprenant les étapes suivantes :
- on part du composé (A) de formule suivante : 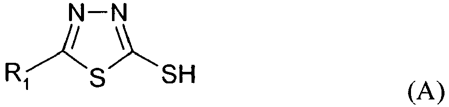 où R₁ est tel que défini précédemment,
- on fait réagir le composé (A) avec un composé de formule R₂NH₂, où R₂ est tel que défini précédemment, en présence d'une base, puis
- on ajoute au milieu réactionnel une composition oxydante comprenant au moins un agent oxydant, pour obtenir le thiadiazole de formule (I).

Selon un premier mode de réalisation, R₁ représente l'hydrogène.

Selon un second mode de réalisation, R₁ représente un groupe méthyle.

De préférence, R₂ représente un groupe cyclohexyle.

Lorsque l'on fait réagir le composé (A) en présence d'une base, celle-ci peut être par exemple une solution aqueuse d'hydroxyde de sodium.

Comme expliqué précédemment, le procédé comprend une étape d'ajout d'une composition oxydante. L'agent oxydant peut être choisi parmi les oxydants classiques tels que le brome, le chlore, l'iode ou encore les acides hypobromique, hypochlorique ou hypoiodique ou bien encore les sels alcalins des acides précédents. Généralement, une solution aqueuse d'hypochlorite de sodium est préférée.

Le système de vulcanisation de la composition selon l'invention peut également comprendre un ou plusieurs accélérateurs primaires additionnels, en particulier les composés de la famille des thiurames, les dérivés dithiocarbamates de zinc ou les thiophosphates.

### II-4. Additifs divers

La composition de caoutchouc selon l'invention peut comporter également tout ou partie des additifs usuels habituellement utilisés dans les compositions d'élastomères destinées à la fabrication de pneumatiques, en particulier de bandes de roulement, comme par exemple des plastifiants ou des huiles d'extension, que ces derniers soient de nature aromatique ou non-aromatique, des pigments, des agents de protection tels que cires anti-ozone (telle que la Cire Ozone C32 ST), anti-ozonants chimiques, anti-oxydants (tel que la 6-paraphénylènediamine), des agents anti-fatigue, des résines renforçantes, des accepteurs (par exemple résine phénolique novolaque) ou des donneurs de méthylène (par exemple HMT ou H3M) tels que décrits par exemple dans la demande WO 02/10269.

De préférence, la composition selon l'invention comporte, à titre d'agent plastifiant préférentiel non aromatique ou très faiblement aromatique, au moins un composé choisi dans le groupe constitué par les huiles naphténiques, paraffiniques, huiles MES, huiles TDAE, les esters (en particulier trioléates) de glycérol, les résines plastifiantes hydrocarbonées présentant une haute Tg de préférence supérieure à 30°C, et les mélanges de tels composés.

La composition selon l'invention peut également contenir, en complément des agents de couplage, des activateurs de couplage de la charge inorganique renforçante ou plus généralement des agents d'aide à la mise en oeuvre susceptibles de manière connue, grâce à une amélioration de la dispersion de la charge inorganique dans la matrice de caoutchouc et à un abaissement de la viscosité des compositions, d'améliorer leur faculté de mise en oeuvre à l'état cru, ces agents étant par exemple des silanes hydrolysables tels que des alkylalkoxysilanes (notamment des alkyltriéthoxysilanes), des polyols, des polyéthers (par exemple des polyéthylèneglycols), des amines primaires, secondaires ou tertiaires (par exemple des trialcanol-amines), des POS hydroxylés ou hydrolysables, par exemple des α,ω-dihydroxy-polyorganosiloxanes (notamment des α,ω-dihydroxy-polydiméthylsiloxanes), des acides gras comme par exemple l'acide stéarique.

### II-5. Fabrication des compositions de caoutchouc

La composition de caoutchouc selon l'invention est fabriquée dans des mélangeurs appropriés, en utilisant deux phases de préparation successives selon une procédure générale bien connue de l'homme du métier : une première phase de travail ou malaxage thermo-mécanique (parfois qualifiée de phase "non-productive") à haute température, jusqu'à une température maximale comprise entre 130°C et 200°C, de préférence entre 145°C et 185°C, suivie d'une seconde phase de travail mécanique (parfois qualifiée de phase "productive") à plus basse température, typiquement inférieure à 120°C, par exemple entre 60°C et 100°C, phase de finition au cours de laquelle est incorporé le système de réticulation ou vulcanisation.

Selon un mode de réalisation préférentiel de l'invention, tous les constituants de base de la composition de l'invention, à l'exception du système de vulcanisation, à savoir la ou les charges renforçantes, l'agent de couplage le cas échéant, sont incorporés de manière intime, par malaxage, à l'élastomère diénique ou aux élastomères diéniques au cours de la première phase dite non-productive, c'est-à-dire que l'on introduit dans le mélangeur et que l'on malaxe thermomécaniquement, en une ou plusieurs étapes, au moins ces différents constituants de base jusqu'à atteindre la température maximale comprise entre 130°C et 200°C, de préférence comprise entre 145°C et 185°C.

A titre d'exemple, la première phase (non-productive) est conduite en une seule étape thermomécanique au cours de laquelle on introduit, dans un mélangeur approprié tel qu'un mélangeur interne usuel, tous les constituants nécessaires, les éventuels agents de mise en oeuvre complémentaires et autres additifs divers, à l'exception du système de vulcanisation. La durée totale du malaxage, dans cette phase non-productive, est de préférence comprise entre 1 et 15 min. Après refroidissement du mélange ainsi obtenu au cours de la première phase non-productive, on incorpore alors le système de vulcanisation à basse température, généralement dans un mélangeur externe tel qu'un mélangeur à cylindres; le tout est alors mélangé (phase productive) pendant quelques minutes, par exemple entre 2 et 15 min.

La composition finale ainsi obtenue est ensuite calandrée par exemple sous la forme d'une feuille ou d'une plaque, notamment pour une caractérisation au laboratoire, ou encore extrudée sous la forme d'un profilé de caoutchouc utilisable par exemple comme une bande de roulement de pneumatique pour véhicule tourisme.

### III. Exemples de réalisation de l'invention

Dans les exemples qui suivent, l'invention est mise en oeuvre avec la N-cyclohexyl-S-(1,3,4-thiadiazol-2-yl)thiohydroxylamine (composé B) de formule suivante :

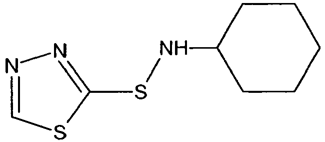

### III-1. Synthèse du composé thiadiazole B

La préparation de ce composé est effectuée à partir du 1,3,4-thiadiazole-2-thiol et de la cyclohexylamine, selon le schéma de synthèse suivant :

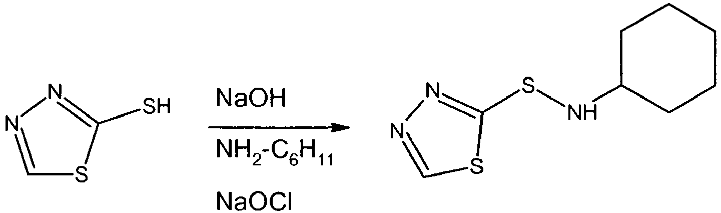

Le 1,3,4-thiadiazole-2-thiol est commercial (numéro CAS [18686-82-3]. Il peut être obtenu à partir de disulfure de carbone et d'hydrate d'hydrazine selon des procédures décrites dans les documents suivants :
1. CH 563 380 (1971)
2. FR 71 47384 (1972)

Le schéma réactionnel de la préparation du 1,3,4-thiadiazole-2-thiol est le suivant :

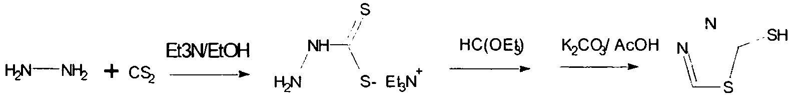

A une solution de 1,3,4-thiadiazole-2-thiol (18,44 g, 0,16 mol) et d'hydroxyde de sodium (14,04 g, 0,35 mol) dans l'eau (900 mL) est ajouté la cyclohexylamine (77,35 g, 0,78 mol). Le mélange est refroidi jusqu'à 0 - 5°C puis est ajoutée goutte à goutte la solution aqueuse de NaOCl (4 % de chlore actif) (343 mL) pendant 15 minutes. La température du milieu réactionnel est maintenue entre 0 et + 5°C. Le milieu réactionnel est ensuite agité pendant une à une heure et demi à une température comprise entre 0 et 5°C.

De l'éther de pétrole (100 mL) est ajouté et le milieu réactionnel est ensuite agité pendant 15 à 30 minutes à une température comprise entre 0 et -4°C. Le précipité est filtré et lavé par de l'eau (200 mL) et de l'éther de pétrole (50 mL) et puis séché pendant 2 à 3 heures sous pression réduite et 12 heures à température ambiante.

Un solide blanc (11,0 g, 0,05 mol) de point de fusion 81-83 °C est obtenu.

La pureté molaire est supérieure à 96 % (RMN ¹H).

La caractérisation complète du produit est réalisée par RMN. Les déplacements chimiques obtenus par RMN 1 H dans l'acétone-d6 sont donnés dans le tableau ci-dessous. La calibration est réalisée sur l'acétone (1,98 ppm en ¹H)

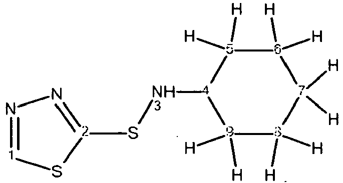

| | 1 | 2 | 3 | 4 | 5 | 6 | 7 | 8 | 9 |
|---|---|---|---|---|---|---|---|---|---|
| δ¹H (ppm) | | | | | 1,98 | 1,67 | 1,52 | 1,67 | 1,98 |
| | 9,21 | - | 4,72 | 2,81 | 1,19 | 1,25 | 1,14 | 1,25 | 1,19 |

### III-2. Préparation des compositions

On procède pour les essais qui suivent de la manière suivante: on introduit dans un mélangeur interne, rempli à 70% et dont la température initiale de cuve est d'environ 90°C, le ou les élastomères diéniques, la ou les charges renforçantes, l'agent de couplage éventuel puis, après une à deux minutes de malaxage, les divers autres ingrédients à l'exception du système de vulcanisation. On conduit alors un travail thermomécanique (phase non-productive) en une étape (durée totale du malaxage égale à environ 5 min), jusqu'à atteindre une température maximale de "tombée" d'environ 165°C. On récupère le mélange ainsi obtenu, on le refroidit puis on ajoute le système de vulcanisation (soufre et composé thiadiazole) sur un mélangeur externe (homo-finisseur) à 70°C, en mélangeant le tout (phase productive) pendant environ 5 à 6 min.

Les compositions ainsi obtenues sont ensuite calandrées soit sous la forme de plaques (épaisseur de 2 à 3 mm) ou de feuilles fines de caoutchouc pour la mesure de leurs propriétés physiques ou mécaniques, soit sous la forme de profilés utilisables directement, après découpage et/ou assemblage aux dimensions souhaitées, par exemple comme produits semi-finis pour pneumatiques, en particulier comme bandes de roulement de pneumatiques.

### III-3. Essais de caractérisation - Résultats

L'objet de cet exemple est de comparer les propriétés rhéométriques d'une composition de caoutchouc utilisable pour la fabrication d'une bande de roulement de pneumatique, comprenant la N-cyclohexyl-S-(1,3,4-thiadiazol-2-yl)thiohydroxylamine (composé B) à titre d'accélérateur primaire de vulcanisation (composition 2) avec les propriétés d'une composition de caoutchouc comprenant la N-cyclohexyl-2-benzothiazyle sulfénamide (« CBS ») (composition 1).

Les formulations des compositions sont données dans le tableau 1. Les quantités sont exprimées en parties pour 100 parties en poids d'élastomére (pce).

**Tableau 1**

| | Composition 1 | Composition 2 |
|---|---|---|
| NR (1) | 100 | 100 |
| N220 (2) | 47,5 | 47,5 |
| Paraffine | 1 | 1 |
| TMQ (3) | 1 | 1 |
| 6-PPD (4) | 1,5 | 1,5 |
| Acide stéarique | 2,5 | 2,5 |
| ZnO | 2,7 | 2,7 |
| Soufre | 1,5 | 1,5 |
| Accélérateur de vulcanisation | 0,6 * | 0,51 ** |

| | | |
|---|---|---|
| * CBS (« Santocure CBS » de la société Flexsys) ** N-cyclohexyl-S-(1,3,4-thiadiazol-2-yl)thiohydroxylamine (1) caoutchouc naturel (2) noir de carbone (3) TMQ :2,2,4-triméthyl-1,2-dihydroquinoline commercialisé par la société Flexys (4) Agent anti-oxydant 6-para-phénylènediamine | | |

La composition de caoutchouc 2 comprenant la N-cyclohexyl-S-(1,3,4-thiadiazol-2-yl)thiohydroxylamine est identique à la composition 1, étant entendu que la CBS est remplacée par une quantité isomolaire de N-cyclohexyl-S-(1,3,4-thiadiazol-2-yl)thiohydroxylamine.

Les propriétés rhéométriques à 150°C sont données dans le tableau 2.

**Tableau 2**

| | Composition 1 (CBS) | Composition 2 (composé B) |
|---|---|---|
| Prop.rhéo. *(DIN)* | 150°C | |
| Δcouple(dN.m) | 7,0 | 6,3 |
| k(min⁻¹) | 0,323 | 0,208 |
| t₀(min) | 4,9 | 3,2 |
| t₉₉ (min) | 19,2 | 25,3 |

Les propriétés rhéométriques obtenues pour la composition contenant la N-cyclohexyl-S-(1,3,4-thiadiazol-2-yl)thiohydroxylamine sont équivalentes à celles obtenues pour la composition contenant la CBS. L'utilisation de la N-cyclohexyl-S-(1,3,4-thiadiazol-2-yl)thiohydroxylamine permet d'obtenir un compromis sur les différentes propriétés rhéométriques.

On note, par ailleurs que le composé B, ainsi que les composés de formule (I) en général, remplacent avantageusement vis-à-vis de l'impact environnemental, les sulfénamides à noyau mercaptobenzothiazole, en ne générant pas contrairement à ces derniers, de mercaptobenzothiazole en se décomposant au cours de la cuisson.

## Revendications

1. Composition de caoutchouc pour la fabrication de pneumatiques, à base d'un ou plusieurs élastomères diéniques, d'une ou plusieurs charges renforçantes et d'un système de vulcanisation, **caractérisée en ce que** ledit système de vulcanisation comprend un ou plusieurs composés thiadiazoles de formule 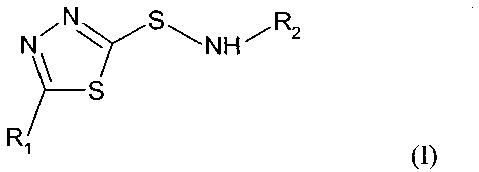 où
R₁ représente H ou un groupe hydrocarboné en C₁-C₂₅ choisi parmi les groupes alkyles linéaires, ramifiés ou cycliques, et les groupes aryles, éventuellement interrompus par un ou plusieurs hétéroatomes,
R₂ représente :
- un groupe alkyle en C₁-C₂₅, linéaire ou ramifié, éventuellement interrompu par un ou plusieurs hétéroatomes, éventuellement substitué par un ou plusieurs groupes alkyles cycliques en C₃-C₁₀ ou aryles en C₆-C₁₂, ou
- un groupe alkyle cyclique en C₁-C₁₀, éventuellement interrompu par un ou plusieurs hétéroatomes, éventuellement substitué par un ou plusieurs groupes alkyles linéaires, ramifiés ou cycliques en C₁-C₂₅ ou aryles en C₆-C₁₂, éventuellement interrompus par un ou plusieurs hétéroatomes.

2. Composition selon la revendication 1 **caractérisée en ce que** R₁ représente H.

3. Composition selon la revendication 2 **caractérisée en ce que** R₁ représente un groupe méthyle.

4. Composition selon l'une quelconque des revendications précédentes **caractérisée en ce que** R₂ représente un groupe cyclohexyle ou un groupe tertbutyle.

5. Composition selon l'une quelconque des revendications précédentes **caractérisée en ce que** le ou lesdits composés thiadiazoles représentent de 0,1 à 7 pce, de préférence de 0,5 à 7 pce, mieux de 0,5 à 5 pce (parties en poids pour cent d'élastomère diénique).

6. Composition selon l'une quelconque des revendications précédentes **caractérisée en ce que** le ou les élastomères diéniques sont choisis dans le groupe constitué par les polybutadiènes, le caoutchouc naturel, les polyisoprènes de synthèse, les copolymères de butadiène, les copolymères d'isoprène et les mélanges de ces élastomères.

7. Composition selon la revendication 6 **caractérisée en ce que** l'élastomère diénique est le caoutchouc naturel.

8. Composition selon l'une quelconque des revendications précédentes **caractérisée en ce que** la ou les charges renforçantes sont choisies parmi la silice, le noir de carbone et leurs mélanges, de préférence le noir de carbone.

9. Composition selon l'une quelconque des revendications précédentes **caractérisée en ce que** la ou les charges renforçantes sont présentes à un taux compris entre 20 et 200 pce, de préférence entre 30 et 150 pce.

10. Procédé pour préparer une composition de caoutchouc pour la fabrication de pneumatiques telle que définie à l'une quelconque des revendications précédentes **caractérisé en ce qu'**il comprend les étapes suivantes :
- incorporer à ou aux élastomères diéniques, au cours d'une première étape dite « non-productive », la ou les charges renforçantes, en malaxant thermomécaniquement le tout, en une ou plusieurs fois, jusqu'à atteindre une température maximale comprise entre 130°C et 200°C,
- refroidir l'ensemble à une température inférieure à 100°C,
- incorporer ensuite, au cours d'une seconde étape, dite
« productive », le système de vulcanisation, puis
- malaxer le tout jusqu'à une température maximale inférieure à 120°C.

11. Utilisation d'une composition selon l'une quelconque des revendications 1 à 9, pour la fabrication d'un article fini ou d'un produit semi-fini destiné à un système de liaison au sol de véhicule automobile.

12. Article fini ou produit semi-fini destiné à un système de liaison au sol de véhicule automobile, comprenant une composition selon l'une quelconque des revendications 1 à 9.

13. Pneumatique comprenant une composition de caoutchouc telle que définie à l'une quelconque des revendications 1 à 9.

14. Utilisation comme accélérateur de vulcanisation dans une composition à base d'un ou plusieurs élastomères diéniques, d'une ou plusieurs charges renforçantes et d'un système de vulcanisation, d'un ou plusieurs composés thiadiazoles de formule (I) tels que définis dans l'une quelconque des revendications 1 à 4.

## Patentansprüche

1. Kautschukzusammensetzung zur Herstellung von Reifen auf der Basis eines oder mehrerer Dienelastomere, eines oder mehrerer verstärkender Füllstoffe und eines Vulkanisationssystems, **dadurch gekennzeichnet, dass** das Vulkanisationssystem eine oder mehrere Thiadiazolverbindungen der Formel 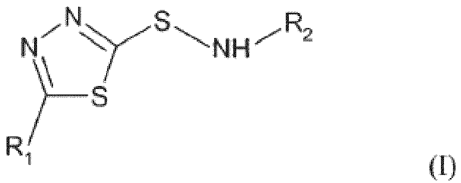 umfasst, wobei
R₁ für H oder eine C₁-C₂₅-Kohlenwasserstoffgruppe, die aus linearen, verzweigten oder cyclischen Alkylgruppen und Arylgruppen, die gegebenenfalls durch ein oder mehrere Heteroatome unterbrochen sind, ausgewählt ist, steht,
R₂ für:
- eine lineare oder verzweigte C₁-C₂₅-Alkylgruppe, die gegebenenfalls durch ein oder mehrere Heteroatome unterbrochen ist und gegebenenfalls durch eine oder mehrere C₃-C₁₀-Cycloalkyl- oder C₆-C₁₂-Arylgruppen substituiert ist, oder
- eine C₃-C₁₀-Cycloalkylgruppe, die gegebenenfalls durch einen oder mehrere Heteroatome unterbrochen ist und gegebenenfalls durch eine oder mehrere lineare oder verzweigte oder cyclische C₁-C₂₅-Alkyl- oder C₆-C₁₂-Arylgruppen, die gegebenenfalls durch ein oder mehrere Heteroatome unterbrochen sind, ausgewählt ist, substituiert ist,
steht.

2. Zusammensetzung nach Anspruch 1, **dadurch gekennzeichnet, dass** R₁ für H steht.

3. Zusammensetzung nach Anspruch 2, **dadurch gekennzeichnet, dass** R₁ für eine Methylgruppe steht.

4. Zusammensetzung nach einem der vorhergehenden Ansprüche, **dadurch gekennzeichnet, dass** R₂ für eine Cyclohexylgruppe oder eine tert.-Butylgruppe steht.

5. Zusammensetzung nach einem der vorhergehenden Ansprüche, **dadurch gekennzeichnet, dass** die Thiadiazolverbindung bzw. die Thiadiazolverbindungen 0,1 bis 7 phe, vorzugsweise 0,5 bis 7 phe und noch besser 0,5 bis 5 phe (Gewichtsteile pro hundert Teile Dienelastomer) ausmacht bzw. ausmachen.

6. Zusammensetzung nach einem der vorhergehenden Ansprüche, **dadurch gekennzeichnet, dass** das Dienelastomer bzw. die Dienelastomere aus der Gruppe bestehend aus Polybutadienen, Naturkautschuk, synthetischen Polyisoprenen, Butadien-Copolymeren, Isopren-Copolymeren und Mischungen dieser Elastomere ausgewählt ist bzw. sind.

7. Zusammensetzung nach Anspruch 6, **dadurch gekennzeichnet, dass** es sich bei dem Dienelastomer um Naturkautschuk handelt.

8. Zusammensetzung nach einem der vorhergehenden Ansprüche, **dadurch gekennzeichnet, dass** der verstärkende Füllstoff bzw. die verstärkenden Füllstoffe aus Kieselsäure, Ruß und Mischungen davon, vorzugsweise Ruß, ausgewählt ist bzw. sind.

9. Zusammensetzung nach einem der vorhergehenden Ansprüche, **dadurch gekennzeichnet, dass** der verstärkende Füllstoff bzw. die verstärkenden Füllstoffe in einer Menge zwischen 20 und 200 phe und vorzugsweise zwischen 30 und 150 phe vorliegt bzw. vorliegen.

10. Verfahren zur Herstellung einer Kautschukzusammensetzung zur Herstellung von Reifen gemäß einem der vorhergehenden Ansprüche, **dadurch gekennzeichnet, dass** man:
- im Lauf eines ersten, als "nichtproduktiv" bezeichneten Schritts den verstärkenden Füllstoff bzw. die verstärkenden Füllstoffe in das Dienelastomer bzw. die Dienelastomere einarbeitet, indem man das Ganze ein- oder mehrmals thermomechanisch knetet, bis eine Höchsttemperatur zwischen 130°C und 200°C erreicht ist;
- die Masse auf eine Temperatur unter 100°C abkühlt;
- dann im Lauf eines zweiten, als "produktiv" bezeichneten Schritts das Vulkanisationssystem einarbeitet und dann
- das Ganze bis zu einer Höchsttemperatur von weniger als 120°C knetet.

11. Verwendung einer Zusammensetzung nach einem der Ansprüche 1 bis 9 zur Herstellung eines Fertigartikels oder Halbzeugs für ein Bodenkontaktsystem eines Kraftfahrzeugs.

12. Fertigartikel oder Halbzeug für ein Bodenkontaktsystem eines Kraftfahrzeugs, umfassend eine Zusammensetzung nach einem der Ansprüche 1 bis 9.

13. Reifen, umfassend eine Kautschukzusammensetzung gemäß einem der Ansprüche 1 bis 9.

14. Verwendung einer oder mehrerer Thiadiazolverbindungen der Formel (I) gemäß einem der Ansprüche 1 bis 4 als Vulkanisationsbeschleuniger in einer Zusammensetzung auf der Basis eines oder mehrerer Dienelastomere, eines oder mehrerer verstärkender Füllstoffe und eines Vulkanisationssystems.

## Claims

1. Rubber composition for the manufacture of tyres, based on one or more diene elastomers, on one or more reinforcing fillers and on a vulcanization system, **characterized in that** the said vulcanization system comprises one or more thiadiazole compounds of formula: 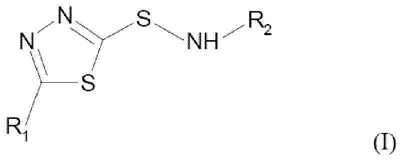 where
R₁ represents H or a C₁-C₂₅-hydrocarbon group chosen from linear, branched or cyclic alkyl groups and aryl groups, optionally interrupted by one or more heteroatoms,
R₂ represents:
- a linear or branched C₁-C₂₅ alkyl group which is optionally interrupted by one or more heteroatoms and which is optionally substituted by one or more cyclic C₃-C₁₀ alkyl or C₆-C₁₂ aryl groups, or
- a cyclic C₃-C₁₀ alkyl group which is optionally interrupted by one or more heteroatoms and which is optionally substituted by one or more linear, branched or cyclic C₁-C₂₅ alkyl or C₆-C₁₂ aryl groups which are optionally interrupted by one or more heteroatoms.

2. Composition according to Claim 1, **characterized in that** R₁ represents H.

3. Composition according to Claim 2, **characterized in that** R₁ represents a methyl group.

4. Composition according to any one of the preceding claims, **characterized in that** R₂ represents a cyclohexyl group or a tert-butyl group.

5. Composition according to any one of the preceding claims, **characterized in that** the said thiadiazole compound or compounds represent from 0.1 to 7 phr, preferably from 0.5 to 7 phr and better still from 0.5 to 5 phr (parts by weight per hundred of diene elastomer).

6. Composition according to any one of the preceding claims, **characterized in that** the diene elastomer or elastomers are chosen from the group consisting of polybutadienes, natural rubber, synthetic polyisoprenes, butadiene copolymers, isoprene copolymers and the mixtures of these elastomers.

7. Composition according to Claim 6, **characterized in that** the diene elastomer is natural rubber.

8. Composition according to any one of the preceding claims, **characterized in that** the reinforcing filler or fillers are chosen from silica, carbon black and their mixtures, preferably carbon black.

9. Composition according to any one of the preceding claims, **characterized in that** the reinforcing filler or fillers are present at a level of between 20 and 200 phr, preferably between 30 and 150 phr.

10. Process for preparing a rubber composition for the manufacture of tyres as defined in any one of the preceding claims, **characterized in that** it comprises the following stages:
- incorporating the reinforcing filler or fillers in the diene elastomer or elastomers, during a first "non-productive" stage, everything being kneaded thermomechanically, in one or more goes, until a maximum temperature of between 130°C and 200°C is reached,
- cooling the combined mixture to a temperature of less than 100°C,
- subsequently incorporating, during a second "productive" stage, the vulcanization system and then
- kneading everything up to a maximum temperature of less than 120°C.

11. Use of a composition according to any one of Claims 1 to 9 in the manufacture of a finished article or a semi-finished product intended for a motor vehicle ground-contact system.

12. Finished article or semi-finished product intended for a motor vehicle ground-contact system, comprising a composition according to any one of Claims 1 to 9.

13. Tyre, comprising a rubber composition as defined in any one of Claims 1 to 9.

14. Use as vulcanization accelerator, in a composition based on one or more diene elastomers, on one or more reinforcing fillers and on a vulcanization system, of one or more thiadiazole compounds of formula (I) as defined in any one of Claims 1 to 4.
